(19) Europäisches Patentamt

European Patent Office

Office européen des brevets

(11) Veröffentlichungsnummer: **0 213 099**

**A2**

(12) ## EUROPÄISCHE PATENTANMELDUNG

(21) Anmeldenummer: 86850271.7

(22) Anmeldetag: 08.08.86

(51) Int. Cl.⁴: **A61K 37/02** , A61K 31/70 , A61K 33/06 , //(A61K37/02,31:70,33:06)

(30) Priorität: 23.08.85 CH 3651/85

(43) Veröffentlichungstag der Anmeldung: 04.03.87 Patentblatt 87/10

(84) Benannte Vertragsstaaten: AT BE CH DE FR GB IT LI LU NL SE

(71) Anmelder: CEDERROTH NORDIC AKTIEBOLAG P.O. Box 715 S-194 27 Upplands Väsby(SE) Anmelder: INTEX PHARMAZEUTISCHE PRODUKTE AG Lautengartenstrasse 12 CH-4010 Basel(CH)

(72) Erfinder: Wickman, Leif Ymervägen 56 S-182 63 Djursholm(SE) Erfinder: Emödi, Gabriel Passwangstrasse 48 CH-4059 Basel(CH)

(74) Vertreter: Barnieske, Hans Wolfgang c/o H.W. Barnieske Patentbyrä AB P.O. Box 25 Turingegatan 26 S-151 21 Södertälje 1(SE)

(54) **Mittel mit antiphlogistischer, immunstimulierender und zytoprotektiver Wirkung, ihre Herstellung und pharmazeutische Verwendungen.**

㋙ Die Erfindung bezieht sich auf ein Mittel mit antiphlogistischer, immunstimulierender und zytoprotektiver Wirkung, Verfahren zu deren Herstellung sowie verwendbare pharmazeutische Zubereitungen. Dieses Mittel besteht hauptsächlich aus einer Kombination folgender Komponenten

a) Kalzium-und/oder Magnesiumkomplexen von Phytohaemagglutininpolyheteroglykanen pflanzlicher Herkunft enthaltend

-etwa 3 bis 25% Phytohaemagglutinine

-bis zu etwa 60% Polyheteroglykane und

-Kalzium bzw. Magnesium in einem Gehalt von 2,0 bis 10,0%, und

b) einem säureneutralisierenden Mittel mit einem säureneutralisiernden Vormögen von etwa 3 M-mol bis etwa 30 M-mol.

Die vorliegende Erfindung bezieht sich auf Mittel mit antiphlogistischer, immunstimulierender und zytoprotektiver Wirkung, Verfahren zu deren Herstellung sowie verwendbare pharmazeutische Zubereitungen.

Lectine pflanzlichen Ursprunges sind schon seit 100 Jahren bekannt. Nach Definition sind sie Proteine die stark Kohlenhydrate binden, haben aber keine enzymatische Aktivität. Die Benennung Phytohaemagglutinin weist darauf hin, dass die meisten Lectine Erythrozyten agglutinierenden Charakter aufweisen. Ihre Zellwuchs und Zellvermehrung stimulierende Wirkung ist bemerkenswert. Als polyklonale Stimulatoren steigern sie den Lymphozyten-Mitose bei Mensch und Tier, hauptsächlich durch die Stimulation der T Zellen. In vitro kann die Steigerung der Interferon-Produktion bei menschlichen Leukozyten nachgewiesen werden.

Die blastogene Aktivität von Lymphozyten gegen Phytohaemagglutinin wird bei der Untersuchung von Immun-Kompetenz angewendet, so z.B. bei der Diagnose von malignen Tumoren - (Bioscience, 1984, Vol. 34, No. 2, pp 95-98), - (Lancet, 1978 2. p 1275).

Zur biologischen Aktivität der Lectine sind zweiwertige Ionen, insbesonders $Ca^{2+}$ nötig. (J. Mol. Biol. 32 453 (1968)).

Bemerkenswert ist auch die antiphlogistische Wirkung von Lectinen. (Japan J. Pharmacol. 32 139-142 (1982)).

Neben den Proteinen sind die Kohlenhydrate, die Oligo-und Polysacchariden die häufig vorkommende andere grosse Makromolekülen bildende Materialen. Aus der Literatur ist die antiphlogistische Wirkung der Arabino-fuco-glukan-Polymere bekannt. (Chem. Pharm. Bull. 32, 4, 1385-1391 - (1984)).

Die genannten Verbindungen haben aber neben ihren vorteilhaften biologischen Eigenschaften auch einige nachteilige Wirkungen, z.B. unter den Naturstoffen befinden sich Lectine die sehr toxisch sind wiez.B. das Ricin/Ricinus communis Lectin, dessen $LD_{50}$ Wert bei Säugetieren ca 1 mikrogramm/kg beträgt.

Die entzündungshemmende Wirkung der Schleimstoffe mit Polyglukan Inhalt und pflanzlicher Herkunft war schon bekannt, doch sind keine Arzneimittel unter diesen Stoffen bekannt. Die entzündungshemmende Wirkung des Kalzium Ions und anderen mehrwertiger Metallionen ist ebenfalls bekannt, hier können aber Penetrationsprobleme an biologischen Barrieren auftreten.

Ueberraschenderweise wurde von uns festgestellt, dass bei der Herstellung von Phytohaemagglutinin Konzentrat aus besonderen Drogenpflanzen Komplexe zu isolieren sind, die Lectin, Polyheteroglykan und Kalzium bzw. Magnesium enthalten.

Bekannt ist weiterhin -A.J.E. Robert und Mitarbeiter (am. J. Physiol. 245) (Gastrointest. Liver Physiol.)8 (1983) 6113-6121 dass der zytoprotektionistische Effekt auf die salzsäureneutralisierende Wirkung eines Puffers zurückgeht. Beschrieben wird das durch Salzsäure und Aethanol induzierte Ulkus und dessen Hemmung durch zwei schon bekannte Substanzen, nämlich Cimetidin und Ranitidin.

Diese Wasserstoff Rezeptoren blockierenden Wirkstoffe zeigen aber mehrere unerwünschte Nebeneffekte (Adverse Drug React. Bull1980, Aug. 300).

Bekannt sind auch neutralisierende Puffer, bzw. säureneutralisierende efferveszente Brausepulver.

Bekannt sind sogenannte Brausepulver mit beispielsweise folgender Zusammensetzung:

Mononatriumzitrat 2000 mg

Natriumbikarbonat 730 mg

Kaliumbikarbonat 690 mg

Natriumkarbonat 160 mg

Zitronensäure 245 mg

Aerosil 200 2 mg

Die säureneutralisierenden Produkte haben den Vorteil, dass sie eine sofortige oder mindestens eine sehr rasche Wirkung zeigen. Sie sind bei durch zuviel Säure verursachten Magenschaden wie z.B.: Reflux bedingter Oesophagitis sehr wirksam. Ergeben aber als Nachteil eine pH-Erhöhung der Magensäure und infolge erniedrigter Alkalireserve kann es zur Alkalose kommen.

Die neutralisierende Reaktion verursacht in manchen Fällen unter anderem eine hyperosmotische Irritation und weiterhin kann bei grösseren Mängen die Antacid-Therapie hemmend auf die Absorption von Antibiotika wirken.

D.W. Piper, I Kang Drugs 1979, 17, 124; M.P. Dutro und A.B. Amerson Br. Med. J. 1981, 305.

Ein Teil der nicht-steoride enthaltenden antiphlogistischen Substanzen beeinflussen die Prostaglandinsynthese. Man hat entdeckt, dass diese Hemmung der Prostaglandin synthese eine Zytoprotektion bewirkt. Einen solchen Effekt wurde auch bei solchen Antiulkuswirkung zeigenden Substanzen gefunden, die nicht über den Prostaglandinmechanismus wirksam sind, wie Sucralfat.

(Sucralfat I.N. Marks, Drugs 1980, 20, 283).

Der therapeutische Effekt von Sucralfat lässt sich durch seine Nebenwirkungen erklären. Die Bildung von Proteinkomplexen hemmt die $H_2$-Rückresorption.

(R. Nagashima, T. Hirano, Arzneimittel Forsch. 1980 , 30, 80).

Die Lectine besitzen wichtige zytoprotektionistische und entzündungshemmende, therapeutische Eigenschaften.

Zweck vorliegender Erfindung ist nun die Schaffung einer Wirkstoffskombination die durch übermässige Magensäureproduktion verursachte Reizung der Magenschleimhaut hemmt, d.h. erfindungsgemäss erfolgt eine Zytoprotektion wobei die Komponenten der erfindungsgemässen Kombination in synergistischer Weise wirken. Desweiteren zeichnen sich die erfindungsgemäss vorgeschlagenen Mittel im Vergleich zu ähnlichen bekannten Substanzen durch sehr niedrige Toxizität aus.

Erfindungsgemäss wird dies durch ein Mittel erreicht, dass aus folgender Kombination besteht

a) Kalzium-und/oder Magnesiumkomplexen von Phytohaemagglutininpolyheteroglykanen pflanzlicher Herkunft enthaltend

-etwa 3 bis 25% Phytohaemagglutinine

-bis zu etwa 60% Polyheteroglykane und

-Kalzium beziehungsweise Magnesium in einem Gehalt von 2,0 bis 10,0%, und

b) einem säureneutralisierenden Mittel mit einem säureneutralisierendem Vermögen von etwa 3 M-mol bis etwa 30 M-mol.

Die erfindungsgemäss vorgeschlagene Kombination der Komponenten a) und b) ist durch eine vorteilhafte, wirksame und zusätzliche Säureneutralisation gekennzeichnet. Gleichzeitig wird durch dieses Mittel eine zytoprotektische Wirkung entfaltet, d.h. da diese Kombination einen sofortigen und auf lange Zeit hin zu beobachtenden therapeutischen Effekt bewirkt.

Zusammenfassend kann somit festgestellt werden, dass die Lectine pflanzlichen Ursprungs langdauernde therapeutische Wirkungen ausüben -im allgemeinen aber keinen direkten Wirkungseffekt zeigen, und dies besonders bei der Refluxoesophagitis.

Die zytoprotektive Wirkung beim Robert-Test, bei welchem als irritierendes Agens Salzsäure-Aethanol eingesetzt wurde, zeigt eindeutig, dass der Säureneutralisierungseffekt für den Patienten eine direkte Linderung der Beschwerden bringt.

Nach der Erfindung wurde somit ein säureneutralisierendes Mittel mit Puffereigenschaften mit einer zweiten Komponente kombiniert, welcher zytoprotektive Wirkung zukommt, d.h. von beiden Komponenten wurden die vorteilhaften Wirkungen übernommen. Die hierdurch erreichte sofortige Wirkung durch den Puffereffekt und die zytoprotektive Eigenschaft ergiebt dem erfindungsgemäss vorgeschlagenen Mittel auch einen auf lange Dauer wirksamen Schutzeffekt.

Nach einer zweckmässigen Ausführungsform der Erfindung beträgt der Anteil der Komponente a) in der Kombination wenigstens 0,1 Gewichtsprozent. Dieser Anteil kann für gewisse Verwendungen beispielsweise bis zu 35 Gewichtsprozent betragen und liegt vorzugsweise zwischen 2-20 Gewichtsprozent.

Zweckmässig kann erfindungsgemäss der Phytohaemagglutinin-polyheteroglykan-Anteil der Komponente a) aus einer oder mehreren Pflanzen der folgenden Familien herrührt: Compositae, Malvaceae, Cucurbitaceae und Gramineae, beispielsweise Tussilago farfara, Althea officinalis Cucurbita pepo convar. Styriaca und Triticum vulgare.

Nach einer vorgezogenen Ausführungsform der Erfindung ist die Komponente a) der Kombination wie folgt gekennzeichnet durch

-ein Molekulargewicht der Phytohaemagglutinine zwischen 10'000 und 75'000 Dalton (bestimmt durch Gelelektrophorese),

-ein Infrarotspektrum gemäss Fig. 2, mit breiten Banden bei 3400 und 1750 cm$^{-1}$ und einer - schmalen Bande bei 1600 cm$^{-1}$

-ein nicht charakteristisches Ultraviolettspektrum,

-einen Gehalt an Calcium bzw. Magnesium von 2,0 bis 10,0 %,

-einen Phosphorgehalt von 0,2 bis 2 %,

-einen Gehalt an Glykanen von bis zu etwa 60 %, darunter Monosaccharide, welche zu mindestens 50 % aus Galaktose und 10 % aus Uronsäuren bestehen,

-eine Elementaranalyse mit 35 bis 45 % Kohlenstoff und 4 bis 7 % Wasserstoff,

-nach der Verbrennung eine Asche folgender Zusammensetzung: 15 bis 25 % Kalium, 0,1 bis 2,0 % Natrium, bis 45 % Kalzium bzw. 5 bis 15 % Magnesium, und 3 bis 6 % Phosphor,

-einen Glührückstand von mindestens 15 % (600 °C, 2 Stunden),

-einen Peptidgehalt von 3 bis 25 % (bestimmt durch Aminosäureanalyse),

-einen Peptidgehalt von 6 bis 30 % (bestimmt durch die Biuret-Reaktion),

-einen Peptidgehalt, welcher zu mindestens 75 % aus folgenden Aminosäuren besteht: Asparaginsäure oder Asparagin, Glutaminsäure oder Glutamin, Alanin, Glycin, Lysin, Serin, Valin und Leucin,

-ein Verhältnis der sauren und der basischen Aminosäuren von mindestens 3:1,

-ein Verhältnis des Peptid-und des Glykangehaltes von etwa 1:10, sowie

-eine zytoprotektive Wirkung bei der Dosis 400 mg/kg per os bei der Ratte von mindestens 40 % - (bestimmt durch den Alkohol-Ulcustest nach Robert).

Erfindungsgemäss besteht die säureneutralisierende Komponente b der Kombination aus einem Fruchtsalz, wie beispielsweise Weinsäure, Zitronensäure, Äpfelsäure oder Fumarsäure, eine Mischung hiervon und Bikarbonaten, wie Natrium-oder Kaliumbikarbonat oder eine Mischung hiervon.

Als säureneutralisierende Komponente b können jedoch auch Magnesiumsalze, wie Magnesiumoxyd, Magnesiumhydroxyd und/oder Magnesiumkarbonat verwendet werden, wobei diese Mischungen zusätzlich auch Kalziumkarbonat enthalten können.

Weiterhin kann erfindungsgemäss die säureneutralisierende Komponente b aus Aluminium-Verbindungen, wie Aluminiumhydroxydgel, Aluminiumphosphatgel oder aus Kombinationen dieser Gele mit Magnesiumsalzen bestehen.

Weitere Merkmale der vorliegenden Erfindung gehen aus den verschiedenen Kennzeichen der Ansprüche hervor.

Die vorliegende Erfindung soll nun anhand nachstehender Beispiele und Ausführungen näher erläutert werden.

Komponente a):

Als Ausgangsmaterial zur Herstellung dieser Komponente eignen sich insbesondere Pflanzen aus den Familien Compositae, Malvaceae, beispielsweise Tussilago farfara, Althea officinalis. Gebraucht werden die entsprechenden Pflanzenteile, nachdem sie fein geschnitten, zerkleinert oder zu einem Brei vermahlen worden sind.

Im Falle der Verwendung von frischen Drogenpflanzen wird die Pflanze nach Mahlen, mit Wasser dessen Menge die 0,8 -1,0fache ist, bezogen auf das Gewicht der Drogenpflanze, vorteilhaft aber mit einer Pufferlösung von einen pH Wert zwischen 8,0 -9,0 z.B. Phosphat-Puffer einmal oder mehrmal behandelt durch Kneten. Nach einem anderen Verfahren kann zu diesem Schritt NaCl Lösung von 0,1 -0,2 Mol/Liter Konzentration verwendet werden. Im Falle der Verwendung von getrockneten Drogenpflanzen wird die Pflanze mit 10 -20% Methanolhaltigem Wasser extrahiert, dessen Menge die 30 -40fache ist, bezogen auf das Gewicht der Drogenpflanze, und dieses Verfahren kann gegebenenfalls mehrmals wiederholt werden.

Die Extraktion bzw. die Behandlung mit Pufferlösung wird bei Raumtemperatur durchgeführt, aber das Erwärmen kann in einigen Fällen vorteilhaft sein, beispielsweise auf etwa 40 -60°C. Während der Extraktion kann Kneten und langsames Rühren kontinuierlich oder diskontinuierlich mit Aufklärung variiert angewendet werden.

Um eine bessere Ausbeute zu erzielen, kann die Extraktion mit dem pflanzlischen Rückstand auf dieselbe Art, wie bereits erfolgt, wiederholt werden. In diesem Fall werden die einzelnen Extrakte für die weitere Aufarbeitung vereinigt.

Nach Abschluss der Extraktion wird der wässrige oder wässrig-alkoholische Extrakt vom verbleibenden festen pflanzlischen Material durch Filtrieren oder Zentrifugieren abgetrennt.

Der Kalzium bzw. Magnesiumkomplex des Phytohaemagglutinin-Polyheteroglykan wird danach aus der Extraktionslösung gefällt, unter Rühren mit Wasser mischbaren Alkohol, vorteilhaft mit Methanol oder Aethanol solcher Menge, dass der Alkoholgehalt der Lösung 75 -95% erreichen soll. Diese Fällungsreaktion wird bei 20 -50°C durchgeführt und zweckmässig wiederholt, so kann reineres Produkt isoliert werden. Nach der Zugabe des Alkohols wird das Rühren beendet, nach 1-24 Std. Sedimentation kann das Präzipitat durch Filtration oder durch Zentrifugieren isoliert werden. Gegebenenfalls kann das Präzipitat mit Alkohol mehrmals gewaschen, dann im Luftstrom oder in Vakuum bei 60°C Temperatur getrocknet werden. So entsteht ein Rohpräparat, was nachstehend auch als Cutiflog bezeichnet wird.

In vielen Fällen ist es zweckmässig das rohe Cutiflog zu reinigen, durch Lösen in einer Pufferlösung, vorteilhaft in einer Phosphat-Pufferlösung deren pH Wert 8,0 -9,0 beträgt und deren Menge 5 -20fache ist, bezogen auf das Gewicht des Präparates. Nach Beendigung des Losungsvorgangs unter Rühren durch Zugabe von feste $(NH_4)_2SO_4$ solcher Menge, dass die Konzentration der Lösung 20 -40% auf $(NH_4)_2SO_4$ erreichen soll, fällt das Lectin-reiche Präparat Cutiflog aus der Lösung, und kann nach 1 -24 Std. Sedimentation durch Filtration oder Zentrifugierung isoliert werden. Das Verfahren kann auch so durchgeführt werden, dass nach dem Lösen im alkalischen Puffer, wird der pH Wert der Lösung durch Zugabe einer geeigneten Säure unter 7,0 zweckmässig zwischen 3,0 -7,0 eingestellt und danach kann die Fällung mit ($NH_4$ / $_2SO_4$) durchgeführt werden.

Die Fällung kann stufenweise ausgeführt werden, so entstehen Cutiflog Präparate mit verschiedenen Lectin-Polyglykan Verhältnissen. Gewünschtenfalls kann die Präzipitation durch leichtes Erwärmen, z.B. auf 20 -50°C beschleunigt werden, auf diese Weise entsteht ein Produkt mit noch reicherem Phytohaemagglutinin.

Auch das rohe Cutiflog Präparat enthält Kalzium und Magnesium Ionen, aber die Menge der Ionen variiert abhängig von der Qualität des Ausgangsmaterials.

Erfindungsgemäss wird der Erdalkaliengehalt des Cutiflog Präparats in einem separaten Gang eingestellt. Ob man rohes oder gereinigtes Cutiflog Präparat als Ausgangsmaterial verwendet, wird es in einem Standard Kalle Dialysis Schlauch - (Hersteller: Kalle AG, BRD-6200, Wiesbaden-Biebrich), zuerst gegen ionenfreies Wasser dialysiert soweit bis sein anorganischer Salzgehalt (d.h. sein Glührückstand) unter 5% fällt. Die Dialyse erfolgt bei Raumtemperatur, in strömendem Wasser, beispielsweise während 10 -48 Std. Ferner wird die äussere Lösung auf 2 -10% $CaCl_2$ bzw. $MgCl_2$ Lösung getauscht, und Dialyse weitergeführt soweit bis im Schlauch das Präparat einen $Ca^{2+}$ oder $Mg^{2+}$ Konzentration von 2 -10% aufweist. Letzte durch die Analyse von einer kleinen Portion des Präparates kontrolliert wird. Diese zweite Dialyse erfolgt auch bei Raumtemperatur und die äussere Lösung strömt in diesem Fall auch, vorteilhaft für 12 -72 Std. Zeitdauer.

Gegebenenfalls kann aber anstatt der zweiten Dialyse eine direkte Reaktion mit Ca bzw. Mg-salze angewendet werden. Die Komplexbildung nun wird mit der Lösung selbst oder mit der auf ein kleines Volumen eingeengten Lösung oder mit dem trockenen, durch Alkoholausfällung oder Gefriertrocknung erhaltenen Produkt ausgeführt. Falls ein festes Produkt erhalten worden ist, wird es zuerst in entmineralisiertem Wasser aufgelöst, was durch Rühren und leichtes Aufwärmen erleichtert wird. Die wässrige Lösung wird dann mit einer wässrigen z.B. 10%-igen Lösung Kalzium oder Magnesiumsalzes behandelt; es eignen sich dazu besonders Kalziumchlorid oder Magnesiumchlorid Lösungen. Zur Bildung des Komplexes wird vorzugsweise eine Zeit lang gerührt.

Der gebildete Komplex wird durch Einengen der Lösung auf ein kleines Volumen und Ausfällen durch einen mit Wasser mischbaren Alkohol, beispielsweise Methanol oder Aethanol als fester Stoff isoliert; zur besseren Ausbildung des Niederschlages kann das Gemisch eine oder zwei Stunden stehengelassen werden. Danach wird der Niederschlag abfiltriert, mit demselben oder einem ähnlichen Alkohol gewaschen und bei leicht erhöhter Temperatur unter normalem Druck getrocknet.

Es wird hier noch auf folgende weitere Herstellungsbeispiele für die Komponente a) verwiesen:

Beispiel 1

1,0 kg Weizenkleie beinhaltend hauptsächlich das Perikarp, Spermoderm und Perisperm von Triticum Vulgare seu Sativum mit einer maximalen Teilchengrösse von 2 mm wird mit 2,4 Liter Wasser, in dem vorher 48 g Kochsalz (NaCl) gelöst worden,waren bei 20°C gut vermischt.

Der entstandene Brei wird 10 Std. lang langsam gerührt und anschliessend mit einer hydraulischen Presse ausgepresst.

Die Pressflüssigkeit beträgt 2150 ml. Sie wird mit 4000 Umdrehungen pro Minute während 30 Minuten zentrifugiert und dann dekantiert.

Es werden 150 g Sediment und 2000 ml Überstehende erhalten. Zur Trennung wird dekantiert und das Überstehende mittels Seitz K5-Filter filtriert.

Das Filtrat wird bei 45°C und im Vakuum von 90 Torr auf ein Volumen von 300 ml eingeengt. Zu diesem Konzentrat werden unter sehr gutem Rühren 900 ml 96 % Aethanol zugegeben. Es entsteht ein Niederschlag, der 24 Std. bei 20°C stehengelassen wird.

Der Niederschlag wird abfiltriert und in 2 mal 50 ml 96 % Aethanol suspendiert, abgesaugt und nochmals mit 50 ml 96 % Aethanol gewaschen. Schliesslich wird er bei 45°C und Normaldruck getrocknet.

Ausbeute : 55 g rohes Extrakt mit folgenden Eigenschaften:

Infrarot Spektrum : bei 3400 bis 1750 $cm^{-1}$, breite Bande

Peptid Gehalt : 15,2 % (Biuret Reaktion)

Asche : 22,9 % (Kalzium-und Magnesiumgehalt 4,5 %)

Glykan Gehalt : 51 % (Gesamtzucker)

Winter (Carrageenin) Test : 40 % Hemmung 10 mg/kg i.p. weibliche Ratten

Robert (Aethanol-Salzsäure Ulkus) Test : 50 % Hemmung 400 mg/kg p.o. weibliche Ratten.

Beispiel 2

Gemäss Beispiel I hergestellt 55 g rohes Cutiflog mit 500 ml Wasser bei 20°C durch 3stündiges Mischen im Turmix ein Brei bereitet und ohne Verzug zentrifugiert.

Das Überstehende, bestehend aus 450 ml wird mit 450 ml n-Butanol geschüttelt und stehengelassen.

Die Butanolphase zeigt eine bräunlich-gelbe Färbung. Das Cutiflog befindet sich unter der flüssigen Phase.

Nochmals wird zentrifugiert, damit alles Butanol entfernt sei.

Zum Niederschlag werden 750 ml Aethanol zugefügt und das Ganze 24 Stunden bei Zimmertemperatur stehengelassen.

Darauf wird filtriert und der Niederschlag 3 mal mit je 40 ml 96 % Aethanol suspendiert, gewaschen und getrocknet.

Ausbeute : 43 g rohes Extrakt von beiger Färbung und foldenden Eigenschaften:

Infrarot Spektrum : bei 3400 bis 1750 cm$^{-1}$ breite Bande

Peptid Gehalt : 18,6 % (mit Biuret Reaktion)

Asche : 17,1 % (Kalzium-und Magnesiumgehalt 2.5 %)

Glykan Gehalt : 60 % (Gesamtzucker)

Winter(Carrageenin) Test : 60 % Hemmung 10 mg/kg i.p. weibliche Ratten

Robert (Aethanol-Salzsäure Ulkus) Test : 65 % Hemmung 400 mg/kg p.o. weibliche Ratten

Beispiel 3

Das Ausgangsmaterial besteht aus 300 ml Konzentrat, hergestellt gemäss Beispiel 1.

Das Überstehende wird abgetrennt und in eine wässrige Lösung enthaltend 150 g Harnstoff und 15 g feingepulvertes Kalziumchlorid (CaCl$_2$) gelöst.

In die so bereitete Lösung wird unter kräftigem Rühren langsam 800 ml 96 % Aethanol zugegeben und das Gemisch 24 Std. lang stehen-gelassen. Darauf wird abgenutscht und 3 mal mit je 30 ml 96 % Aethanol gewaschen. Nach dem Absaugen wird das Gut bei höchstens 45°C getrocknet.

Ausbeute : 45 g rohes Extrakt, beigefabrig mit folgenden Eigenschaften:

Infrarot Spektrum : bei 3400 bis 1750 cm$^{-1}$, Breite Bande

Peptid Gehalt : 14,6 % (mit Biuret Reaktion)

Asche : 19,6%(Kalzium-und Magnesiumgehalt 3,6 %)

Glykan Gehalt : 55 % (Gesamtzucker)

Winter (Carrageenin) Test : 50 % Hemmung 10 mg/kg i.p. weibliche Ratten

Robert (Aethanol-Salzsäure Ulkus) Test : 58 % Hemmung 400 mg/kg p.o. weibliche Ratten

Beispiel 4

Zu 1,0 kg frisch gesammelten Tussilago farfara (Compositae) (Trockensubstanz 20%) werden 560 ml KH$_2$PO$_4$ Pufferlösung von 8,5 pH Wert gegeben, zu einem Brei gemahlen und in einer Knetmaschine 3 Std. lang bei 40°C Temperatur gerührt, nachher zentrifugiert. Der Bodenkörper wird in 300 ml Pufferlösung von einem pH Wert 8,5 suspendiert, und nach einer halben Stunde Rühren zentrifugiert. Der Bodenkörper wird sorgfältig entwässert. Die Filtrate werden vereinigt, das Gemisch wird zum Absetzen des Niederschlages während 4 Std. stehengelassen, und über Seitz K5 Filter filtriert. So gewinnt man 1450 ml klares Filtrat. Das Filtrat wird unter kräftigem Rühren mit 4350 ml Aethanol versetzt, und 8 Std. lang bei Raumtemperatur sedimentiert. Die wässrige Mutterlauge wird vom Präzipitat dekantiert, der Rest wird im 100 ml Wasser aufgenommen und gefriergetrocknet. Die Ausbeute ist 20,0 g rohes Cutiflog.

Peptid Gehalt: 15% (Biuret Reaktion)

Glykan Gehalt: 52 % (Gaschromatographie nach Hydrolyse)

Asche: 24% (bei 600°C, 2 Std. lang erhitzt)

Kalzium: 3,6% (Atomabsorptionsspektroskopie)

Biologische Aktivität: 50% (Carrageen Rattenpfoten Test)

Das erhaltene 20,0 g rohe Cutiflog wird im 400 ml Wasser gelöst, der Lösung werden konzentrierte NH$_4$OH zugegeben bis ihr pH den Wert 9,0 erreicht. Das Gemisch wird während 3 Std. bei 40°C gerührt und der nichtlösbare Rest durch Zentrifugieren separiert. Zur klaren Lösung werden unter Rühren 160 g (NH$_4$)$_2$SO$_4$ zugegeben und das Gemisch zum Absetzen des Niederschlages während 12 Std. bei 25°C stehengelassen. Der entstandene Niederschlag wird durch Zentrifugieren getrennt und durch Verreiben mit 100 ml Azeton entwässert. Das Gewicht der Azeton-nassen Lectin-Fraktion ist 3,0 g. Das Produkt wird in 10 ml 8%-iger wässriger NH₄OH Lösung aufgelöst, die Lösung wird in einem Standard Kalle Dialyseschlauch während 12 Std. gegen strömendes ionenfreies Wasser dialysiert.

Während der Dialyse vermindert sich der anorganische Salzgehalt des Präparates auf 3,0%, was durch eine quantitative Aschenanalyse aus kleinen aliquot Teil kontrolliert werden kann. Nach Erreichen der gewünschten anorganischen Salz Konzentration wird die Dialyse-Lösung mit 10%-iger CaCl₂ Lösung ausgetauscht und die Dialyse während 12 Std. weitergeführt. Das Endprodukt wird zuletzt als gefriergetrocknet isoliert. Die Ausbeute ist 0,50 g gereinigtes Cutiflog.

**Komponente b):**

Die säureneutralisierende Komponente b) kann zweckmässig aus einem Fruchtsalz bestehen.

Eine zweckmässige säureneutralisierende Puffer-Lösung -nachstehend auch als Samarin bezeichnet wurde wie folgt hergestellt:

Natriumbikarbonat 52 %

Weinsäure 44 %

Kalium/Natriumtartrat 1 %

wobei die wässrige Lösung der obigen Komponente ein pH-Wert von etwa 5,0 aufweist.

Eine weitere zweckmässige Zusammensetzung II der Komponente b) ist wie folgt:

Natriumbikarbonat 2,08 g

Weinsäure 1,76 g

Natriumkarbonat 0,12 g

Kalium-Natriumtartrat 0,04 g

Die Komponente b) kann hierüberhinaus auch aus anderen bekannten säure-neutralisierenden Mischungen bestehen und hierzu wird auf die in den beiliegenden Ansprüchen angegebenen Zusammensetzungen verwiesen.

**Erfindungsgemässe Kombination**

0,2 Gewichtsprozent der nach Beispiel 1 hergestellten Komponente a) wurde mit 4,0 g der unter II oben angegebenen Komponente b) gemischt und eine wässrige Lösung der so erhaltenen Pulvermischung in einer Tagesdosis von 0,5 g/Tag verabreicht.

Hierbei konnte festgestellt werden, dass auch eine längere Verabreichung dieser Dosis zu keinen toxischen Nebenwirkungen führte.

Ein erfindungsgemässes Präparat obiger Zusammensetzung wurde auch bei den nachstehenden Versuchen verwendet.

Hierbei wurden gewöhnliche Wistar-Ratten 200 -250 g/p. Ratte in Gruppen von je 10 Tieren während eines Tages ohne Nahrung gehalten und dann durch eine Magensonde einmal mit 1 bis 2 ml wasserfreiem Aethanol, welches 0,5% HCl enthielt, behandelt. 1 Stunde vorher wird das zu prüfende Produkt mit Hilfe einer Magensonde per os verabreicht.

Nach Sektion der Tiere wird im Magen die Länge des entstandenen Geschwüres und die Grösse des entstandenen Schleimhautödems gemessen und mit jenen der mit dem Produkt nicht behandelten Kontrolltieren verglichen.

Festgestellte Hemmung in %

|  | 25 mg/kg p.o. | 50 mg/kg p.o. | 100 mg/kg p.o. | 400 mg/kg p.o. |
|---|---|---|---|---|
| Komponente a) | 0 | 18 | 32 | 58 |
| Komponente b) | 0 | 0 | 28 | 55 |
| Erfindungsgemässe Kombination der Komponenten a) + b) | 34 | 40 | 48 | 66 |

Es ist erwähnenswert, dass bei einer Dosierung der erfindungsgemässen Kombination von 400 mg/kg per os eine Hemmung von 66% erhalten wurde, statt wie erwartet von nur 46,5%.

Das bekannte Mittel Sucralfat wurde im gleichen Test wie oben als Referenzsubstanz verwendet.

Bei der gleichen Dosierung von 400 mg/kg per os wurde nur eine 50%-ige Hemmung erhalten, d.h. mit der Kombination Samarin/Sucralfat oder Cutiflog/Sucralfat konnte kein synergistischer Effekt beobachtet werden.

Erfindungsgemäss hergestellte Kombinationspräparate zeichnen sich durch eine sehr geringe Toxizität aus, die bei einer therapeutischen Verwendung unter 1 per 100 bleibt.

Erfindungsgemäss kann das vorliegende Kombinationspräparat aus einer Mischung von Granulaten der Komponenten a) und b) hergestellt werden, wobei dann die Granulate oxidationssicher verpackt sein sollten.

Das erfindungsgemäss vorgeschlagene Präparat kann zweckmässig profylaktisch wie auch zur Behandlung von gastrointestinalen Krankheiten, wie beispielsweise Gastritis, Ulcus Ventriculi, eingesetzt werden.

**Ansprüche**

1. Mittel mit antiphlogistischer, immunstimulierender und zytoprotektiver Wirkung, gekennzeichnet durch eine Kombination von

    a) Kalzium-und/oder Magnesiumkomplexen von Phytohaemagglutininpolyheteroglykanen pflanzlicher Herkunft enthaltend

-etwa 3 bis 25% Phytohaemagglutinine

-bis zu etwa 60% Polyheteroglykane und

-Kalzium bzw. Magnesium in einem Gehalt von 2,0 bis 10,0%, und

    b) einem säureneutralisierenden Mittel mit einem säureneutralisierenden Vermögen von etwa 3 M-mol bis etwa 30 M-mol.

2. Mittel nach Anspruch 1, dadurch gekennzeichnet, dass der Anteil der Komponente a) in der Kombination wenigstens 0,1 Gewichtsprozent ausmacht.

3. Mittel nach Anspruch 2, dadurch gekennzeichnet, dass der Anteil der Komponente a) 0,1 - 35 Gewichtsprozent, vorzugsweise 2 -30 Gewichtsprozent ausmacht.

4. Mittel nach Anspruch 1, dadurch gekennzeichnet, dass der Phytohaemagglutininpolyheteroglykan-Anteil der Komponente a) aus einer oder mehreren Pflanzen der folgenden Familien herrührt: Compositae, Malvaceae, Cucurbitaceae und Graminae, beispielsweise Tussilago farfara, Althea officinalis, Cucurbita pepo convar. Styriaca und Triticum vulgare.

5. Mittel nach Anspruch 1, dadurch gekennzeichnet, dass das Molekulargewicht der Phytohaemagglutinine der Komponente a) zwischen 10 000 und 75 000 Dalton bestimmt durch Gelelektrophorese ist.

6. Mittel nach Anspruch 1, dadurch gekennzeichnet, dass der Glykanen-Anteil Monosaccharide der Komponente a) welche zumindestens 50% aus Galaktose und 10% aus Uronsäuren bestehen, enthält.

7. Mittel nach Anspruch 1, gekennzeichnet durch einen Peptidgehalt der Komponente a) von 3 bis 25% bestimmt durch Aminosäureanalyse.

8. Mittel nach Anspruch 1, gekennzeichnet durch einen Peptidgehalt der Komponente a) von 6 bis 30% bestimmt durch die Biuret-Reaktion.

9. Mittel nach Anspruch 1, gekennzeichnet durch einen Peptidgehalt der Komponente a), welcher zu mindestens 75% aus folgenden Aminosäuren besteht: Asparaginsäure oder Asparagin, Glutaminsäure oder Glutamin, Alanin, Glycin, Lysin, Serin, Valin und Leucin, wobei das Verhältnis der sauren und der basischen Aminosäuren etwa wenigstens 3:1 ausmacht.

10. Mittel nach Anspruch 1, dadurch gekennzeichnet, dass die Komponente a)

-ein Molekulargewicht der Phytohaemagglutinine zwischen 10 000 und 75 000 Dalton (bestimmt durch Gelelektrophorese),

-ein Infrarotspektrum gemäss Fig. 2 mit breiten Banden bei 3 400 und 1 750 cm⁻¹ und einer - schmalen Bande bei 1 600 cm⁻¹,

-ein nicht charakteristisches Ultraviolettspektrum,

-einen Gehalt an Kalzium bzw. Magnesium von 2,0 bis 10,0%,

-einen Phosphorgehalt von 0,2 bis 2%,

-einen Gehalt an Glykanen von bis zu etwa 60%, darunter Monosaccharide, welche zu mindestens 50% aus Galaktose und 10% aus Uronsäuren bestehen,

-eine Elementaranalyse mit 35 bis 45% Kohlenstoff und 4 bis 7% Wasserstoff,

-nach der Verbrennung eine Asche folgender Zusammensetzung: 15 bis 25% Kalium, 0,1 bis 2,0% Natrium, 20 bis 45% Kalzium bzw. 5 bis 15% Magnesium, und 3 bis 6% Phosphor,

-einen Glührückstand von mindestens 15% (600°C, 2 Stunden),

-einen Peptidgehalt von 3 bis 25% (bestimmt durch Aminosäureanalyse),

-einen Peptidgehalt von 6 bis 30% (bestimmt durch die Biuret-Reaktion),

-einen Peptidgehalt, welcher zu mindestens 75% aus folgenden Aminosäuren besteht: Asparaginsäure oder Asparagin, Glutaminsäure oder Glutamin, Alanin, Glycin, Lysin, Serin, Valin und Leucin,

-ein Verhältnis der sauren und der basischen Aminosäuren von mindestens 3:1,

-ein Verhältnis des Peptid-und des Glykangehaltes von etwa 1:10, sowie

-eine zytoprotektive Wirkung bei der Dosis 400 mg/kg per os bei der Ratte von mindestens 40% - (bestimmt durch den Alkohol-Ulcustest nach Robert)

aufweist.

11. Mittel nach Anspruch 1, dadurch gekennzeichnet, dass die säureneutralisierende Komponente b) der Kombination aus einem Fruchtsalz, wie beispielsweise eine Mischung von Weinsäure, Zitronensäure, Äpfelsäure oder Fumarsäure, und von Bikarbonaten, wie Natrium-oder Kaliumbikarbonat oder eine Mischung hiervon, besteht.

12. Mittel nach Anspruch 1, dadurch gekennzeichnet, dass die säureneutralisierende Komponente b) der Kombination aus Magnesiumsalzen, wie Magnesiumoxyd, Magnesiumhydroxyd und/oder Magnesiumkarbonat besteht.

13. Mittel nach Anspruch 12, dadurch gekennzeichnet, dass die säureneutralisierende Komponente b) der Kombination zusätzlich Kalziumkarbonat enthält.

14. Mittel nach Anspruch 1, dadurch gekennzeichnet, dass die säureneutralisierende Komponente b) der Kombination aus Aluminiumverbindungen, wie Aluminiumhydroxydgel, Aluminiumphosphatgel oder aus Kombinationen dieser Gele mit Magnesiumsalzen besteht.

15. Mittel nach Anspruch 1, dadurch gekennzeichnet, dass die säureneutralisierende Komponente b) aus

438 -622 mg Na

6 -225 mg K

136 -200 mg $HCO_3$ und

1756 -1886 mg Weinsäure

besteht und mit 200 -400 mg der Komponente a) vermischt wird.

16. Mittel nach Anspruch 1, gekennzeichnet durch folgende Zusammensetzung

| | |
|---|---|
| Komponente a) | 0,15 g |
| Komponente b) | |
| bestehend aus | |
| Natriumbikarbonat | 2,08 g |
| Weinsäure | 1,76 g |
| Natriumkarbonat | 0,12 g |
| Kalium-Natriumtartrat | 0,04 g　=　4,00 g |

Nach Auflösung dieser als Trockenpräparat vorliegenden Kombination in wässriger Lösung wird diese Dosis 1 -3 Mal pro Tag verabreicht.